# EUROPEAN PATENT APPLICATION

(11) **EP 0 555 718 A1**
(43) Date of publication of application: **18.08.1993**
(21) Application number: 93101502.8
(22) Date of filing: 10.12.1987
(51) Int. Cl.: A61K 31/47

(54) **Pharmaceutical compositions in the form of soft ampoules of plastics containing a nedocromil solution**

(30) Priority: 23.12.1986 GB 8630767; 23.12.1986 GB 8630769; 24.12.1986 GB 8630904; 20.03.1987 GB 8706684
(62) Divisional of application: 87310882.3
(71) Applicant: FISONS plc, Ipswich Suffolk IP1 1QH (GB)
(72) Inventor: Clark, Andrew Reginald, Half Moon Bay, CA 94019 (US); Ratcliffe, Julia Helena, Alverstoke, Gosport, Hampshire PO12 2DH (GB); Wright, Paul, Bramcote, Nottinghamshire NG9 3FS (GB)
(74) Representative: Jones, Stephen Anthony

(57) **Abstract**

Soft ampoules of plastics material sterile-filled with a unit dose of an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, and sealed, are disclosed.

Also described is the use of the solutions in the treatment of e.g. conjunctivitis, keratitis, 'allergic eyes', adenovirus infections, corneal homograft rejection, anterior uveitis, nasal polyps, vasomotor rhinitis, allergic manifestations of the nasopharynx, reversible obstructive airways disease, Crohn's disease, distal colitis and proctitis.

## Description

This invention relates to novel pharmaceutical compositions, methods for their preparation and methods of treatment using them.

In British Patent No. 2022078 there are disclosed a number of pyranoquinoline compounds which are indicated for use in the treatment of reversible airway obstruction. Pharmaceutical compositions containing these compounds are also described, especially compositions in which the active ingredient is in powder form with a mass median diameter of from 0.1 to 10 µm. British Patent Application No. 2157291A describes the particular utility of the disodium salt of one of these compounds, 9-ethyl-6,9-dihydro-4,6-dioxo-10-propyl-4H-pyrano(3,2-g)-quinoline-2,8-dicarboxylic acid, in the treatment of reversible airway obstruction. Also described are powdered aerosol compositions of this salt for administration to the lung and physical forms of the salt which are especially suitable for formulation in this way.

We have also found that when it is administered in aqueous solution the same compound is efficacious in the treatment of a variety of disorders of the eye, notably conjunctivitis, as well as in the treatment of certain disorders associated with other organs. The solutions are administered by a route appropriate to the condition being treated. For instance, administration may be to the eye, intra-nasally (e.g. as a nasal spray), by inhalation as a nebulised cloud or rectally as an enema.

Conventionally, unit doses of aqueous solutions for use in nebulisers are packed in glass or plastics ampoules which are broken open immediately prior to use. Such packaging is both wasteful and expensive to manufacture. Furthermore, the breaking-open of glass ampoules could lead to the formation of glass shards which can be inhaled with the solution.

We have now found a form of packaging for single-dose solutions which overcomes the above-mentioned disadvantages.

Thus, according to the invention, we provide a soft ampoule of plastics material sterile-filled with a unit dose of an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, and sealed.

We prefer the plastics material to be permeable to carbon dioxide. Thus, when the ampoule is stored, carbon dioxide dissolves in the solution and the pH is lowered. Since the stability of the active ingredient is greater at lower pH, this has the effect of enhancing the stability of the solution.

Suitable plastics materials from which the ampoule may be manufactured include low-density polyethylene.

A plurality of ampoules may be connected to, and formed integrally with, an anchorage member which may be adapted to receive a label or writing, e.g. to identify the contents of the ampoules.

The plastics material may include a pigment or pigments such that the ampoules correspond in colour to the solution contained within them.

Pharmaceutically acceptable salts of the active ingredient include salts with metal cations, such as alkali metal cations. We particularly prefer the disodium salt which is commonly referred to as nedocromil sodium.

The solution may contain from 0.1 to 10% w/v of the active ingredient. However, we prefer the active ingredient to be present at a level of less than 5% and more particularly less than 3% w/v, e.g. 0.5%, 1.0% or 2.0% w/v. The concentration of choice will depend of course inter alia on the nature of the condition to be treated, its severity and the mode of administration of the solution.

In addition to the active ingredient the solution generally contains one or more pharmaceutically acceptable additives. Examples of classes of additive which may be present are:
a) chelating or sequestering agents,
b) preservatives, and
c) viscosity-modifying agents.

Suitable chelating or sequestering agents include sodium carboxymethyl cellulose, citric, tartaric or phosphoric acid, and amino carboxylate compounds. The preferred chelating agent, however, is ethylenediamine tetraacetic acid or a salt thereof, especially its di-sodium salt.

The concentration of the chelating or sequestering agent should be such as to ensure that no precipitate of metal salts of the active ingredient occurs. A suitable concentration of chelating or sequestering agent may be from 0.005 to 0.5, e.g. 0.01 or 0.1% w/v.

As the solution are put up in unit dosage form, preservatives may be incorporated, but are generally not necessary. The choice of preservative, where the solution contains a preservative, may depend on the route of administration. Preservatives suitable for solutions to be administered by one route may possess detrimental properties which preclude their administration by another route. For nasal and ophthalmic solutions, preferred preservatives include quaternary ammonium compounds, in particular the mixture of alkyl benzyl dimethyl ammonium compounds known generically as 'Benzalkonium Chloride'. For solutions to be administered by inhalation, however, the preferred preservative is chlorbutol. Other preservatives which may be used, especially for solutions to be administered rectally, include alkyl esters of p-hydroxybenzoic acid and mixtures thereof, such as the mixture of the methyl, ethyl, propyl and butyl esters sold under the name Nipastat™.

The concentration of preservative should be such as to ensure effective preservation of the solution i.e. such that bacterial growth in the solution is inhibited. For most preservatives the concentration will typically lie in the range 0.001 to 0.1% w/v. However, in the case of chlorbutol acceptable concentrations are greater than 0.25% but less than 0.6% w/w, i.e. the concentration of chlorbutol is 0.25 to 0.6%, preferably 0.3 to 0.55%, e.g. 0.5% w/w.

Suitable viscosity enhancing agents which may be incorporated into the solution include carbomers i.e. polymers of acrylic acid cross-linked with a polyalkenyl polyether, aluminium magnesium silicate, methylcellulose, hydroxypropyl methylcellulose and other cellulose derivatives.

The viscosity of the solution will depend *inter alia* on the particular viscosity enhancing agent used and its molecular weight and on the target organ. However, the solution preferably has a viscosity of at least 100 mPa.s, more preferably at least 200 mPa.s and especially more than 400 mPa.s, at a shear rate of 50 s⁻¹. The viscosity of the solution is preferably less than 10000 mPa.s, more preferably less than 1000 mPa.s and especially less than 500 mPa.s, at a shear rate of 50 s⁻¹.

Viscosities are preferably determined using a rotational viscometer such as a Rheomat™ 30, at a temperature of from 15 to 25°C, e.g. 20°C.

For applications which involve the solution being administered as a spray eg through a nasal pump, we prefer the viscosity enhancing agent to have a low viscosity at high shear rates, e.g. from 100 mPa.s to 300 mPa.s at 140 s⁻¹, and a high viscosity at low shear rates, e.g. from 700 mPa.s to 1200 mPa.s at 15 s⁻¹.

Viscosity enhancing agents which we prefer include hydroxypropyl methylcellulose and carbomers, in particular the carbomer sold as Carbopol™ 934P, the viscosity of a neutralised 0.5% w/w aqueous dispersion of which lies in the range 29400 to 39400 mPa.s and the heavy metal content of which is 0.002% or less.

The amount of viscosity-modifying agent required to achieve the desired viscosity will depend on the particular agent used and also on its molecular weight. However, we prefer to use up to about 2% w/w, e.g. 0.5 to 1.5% w/w, of viscosity enhancing agent.

The solution may also contain other conventional excipients, e.g. sodium chloride, dextrose or mannitol, and buffers, e.g. sodium dihydrogen orthophosphate (sodium acid phosphate BP), di-sodium hydrogen phosphate (sodium phosphate BP) sodium citrate/citric acid, and boric acid/sodium borate. The proportion and concentration of excipients and buffers may be varied within fairly wide ranges, provided the resulting solution is stable and non-irritant when applied to the appropriate tissues. The maximum total concentration of excipients and buffers is preferably less than 5% w/v and more preferably less than 2% w/v. Solutions for rectal administration may contain bulking agents, e.g. methyl cellulose, to aid retention in the bowel.

The solution may be made isotonic with physiological fluids by the incorporation of a suitable tonicity agent e.g. sodium chloride. The solution typically contains from about 0.1 to 1.0, more typically 0.5 to 1.0% w/v, sodium chloride.

Although physiological pH is about 7.4, we prefer the pH of the solution to be in the range 3.5 to 6, preferably 4 to 5.5. In this range of pH, the stability of the solution is enhanced, surprisingly with no deleterious effects such as undue tissue irritation.

The solutions with which the ampoules are filed may be made up, for example, by dissolving the active ingredient, chelating or sequestering agent (if included) and excipients (if included) in freshly distilled water, adding to this solution an aqueous solution containing the preservative (if included), adjusting the pH if necessary, making the solution up to the desired volume with distilled water, stirring and then sterilising. Alternatively, the active ingredient, chelating or sequestering agent (if included) and excipients (if included) may be dissolved in a solution containing the preservative. Sterilisation is preferably performed by sterile filtration into a previously sterilised container. Where the preservative used is benzalkonium chloride, some complex formation may occur when the solutions of active ingredient and preservative are mixed. It may thus be necessary to use a higher concentration of preservative than is desired for the final product.

Aqueous solutions containing active ingredient, a viscosity enhancing agent, e.g. a carbomer, and, optionally, a preservative, e.g. benzalkonium chloride, may be prepared by dispersing the viscosity enhancing agent in an aqueous solution containing the preservative (if used) and the active ingredient, and then, if required, adjusting the pH, e.g. by addition of sodium hydroxide, and, if desired, further diluting the solution with water.

The solution of the preservative and the active ingredient may be made simply by dissolving the ingredients in water which is low in metal ions.

The solution is preferably made up at a temperature of from 10 to 50°C, for example at room temperature.

The solutions may be used for the treatment of the following conditions:
conjunctivitis, keratitis, 'allergic eyes', adenovirus infections, corneal homograft rejection, anterior uveitis;
nasal polyps, vasomotor rhinitis, allergic manifestations of the nasopharynx;
reversible obstructive airways disease;
Crohn's disease, distal colitis and proctitis.

By the term 'conjunctivitis' we mean inflammatory disorders of the conjunctiva commonly characterised by photophobia and irritation. The condition may be bacterial or viral and encompasses a number of specific types of conjunctivitis; for instance, seasonal allergic conjunctivitis, perennial allergic conjunctivitis, vernal catarrh (vernal kerato-conjunctivitis), 'irritable eye' or 'non-specific conjunctivitis', Herpes Simplex Conjunctivitis, Herpes Zoster Conjunctivitis and phlyctenular conjunctivitis.

Similarly, by the term 'keratitis' we mean inflammation of the cornea which may involve its superficial surface ('superficial keratitis' including the localised form known as 'corneal ulceration') or be confined to the deeper layers ('interstitial keratitis'). Other particular forms of keratitis which may be mentioned include Herpes Simplex Keratitis and Herpes Zoster Keratitis.

Proctitis includes chronic (i.e. ulcerative) and non-specific proctitis.

The dosage to be administered will of course vary with the condition to be treated, with its severity and with its location. However, in general for use in the eye a dosage of 1 or 2 drops (e.g. from 0.3 to 1.2 mg of active ingredient depending on the concentration of active ingredient) into the affected eye from 2 to 4 times a day is found to be satisfactory. More frequent dosage may, of course, be used if desired. For use in the nose a dosage of about 0.25 ml (e.g. from 1.2 mg to 5.0 mg of active ingredient) is indicated.

For rectal administration a total daily dosage of from 50 to 1000 mg of active ingredient, more preferably 100 to 400 mg, administered in smaller doses 2 to 4 times a day is found to be satisfactory. A dosage unit may conveniently contain from about 25 to 200 mg of active ingredient.

For administration by inhalation a daily dosage of from 10 mg to 100 mg is, in general, found to be satisfactory. The daily dosage may be administered in divided doses, e.g. from 2 to 4 times a day. More frequent dosage may of course be used if required.

The aqueous solutions are advantageous in that they are longer acting, more acceptable to the patient, give rise to higher concentrations of active ingredient in target tissues, give rise to effective concentrations of active ingredient in target tissues for a longer time or are more stable than known similar formulations.

The invention is illustrated by the following Example.

### Example

| Non-preserved nebuliser solution | |
|---|---|
| Nedocromil Sodium | 0.5 % w/v |
| Sodium Chloride | 0.79 |
| Hydrochloric acid | q.s. |
| Purified Water | to 100 |

Nedocromil sodium (5 g) and sodium chloride (7.9 g) were dissolved in purified water (900 ml). The pH of the solution was adjusted to between 5 and 5.5 by addition of hydrochloric acid and the volume made up to 1000 ml with purified water.

The solution was sterile-filled into low-density polyethylene ampoules which were then sealed.

## Claims

1. A soft ampoule of plastics material sterile-filled with a unit dose of an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, and sealed.

2. A soft ampoule according to Claim 1, wherein the plastics material is permeable to carbon dioxide.

3. A soft ampoule according to Claim 1 or 2, wherein the active ingredient is nedocromil sodium.

4. A soft ampoule according to any one of Claims 1 to 3, wherein the solution has a pH of from 3.5 to 6.

5. A soft ampoule according to Claim 4, wherein the solution has a pH of from 4 to 5.5.

6. A soft ampoule according to any one of the preceding claims, wherein the concentration of active ingredient in the solution is from 0.1 to 5% w/v.

7. A soft ampoule according to any one of the preceding claims, wherein the solution also contains a viscosity-modifying agent.

8. A soft ampoule according to according to Claim 7, wherein the solution has a viscosity of from 100 to 10000 mPa.s at a shear rate of 50 s⁻¹.

## Claims (Claims for the following Contracting State(s): ES)

1. A method for the manufacture of a soft ampoule of plastics material containing a unit dose of an aqueous solution containing, as active ingredient, 9-ethyl-6,9-dihydro-4,6-dioxo-10-propyl-4H-pyrano(3,2-g)quinoline-2,8-dicarboxylic acid or a pharmaceutically acceptable salt thereof, which method comprises sterile-filling the ampoule with the solution and sealing the ampoule.

2. A method according to Claim 1, wherein the plastics material is permeable to carbon dioxide.

3. A method according to Claim 1 or 2, wherein the active ingredient is nedocromil sodium.

4. A method according to any one of Claims 1 to 3, wherein the solution has a pH of from 3.5 to 6.

5. A method according to Claim 4, wherein the solution has a pH of from 4 to 5.5.

6. A method according to any one of the preceding claims, wherein the concentration of active ingredient in the solution is from 0.1 to 5% w/v.

7. A method according to any one of the preceding claims, wherein the solution also contains a viscosity-modifying agent.

8. A method according to according to Claim 7, wherein the solution has a viscosity of from 100 to 10000 mPa.s at a shear rate of 50 s⁻¹.
